Europäisches Patentamt

European Patent Office

Office européen des brevets

(11) Publication number: **0 190 195**
**B1**

## (12) EUROPEAN PATENT SPECIFICATION

(45) Date of publication of patent specification: **09.01.91**

(21) Application number: **85903508.1**

(22) Date of filing: **20.06.85**

(86) International application number:
**PCT/US85/01156**

(87) International publication number:
**WO 86/00234 16.01.86 Gazette 86/02**

(51) Int. Cl.⁵: **A 61 N 1/36**

(54) PROCESS AND APPARATUS FOR DIAPHRAGMIC STIMULATION.

(30) Priority: **21.06.84 FR 8409785**

(43) Date of publication of application:
**13.08.86 Bulletin 86/33**

(45) Publication of the grant of the patent:
**09.01.91 Bulletin 91/02**

(84) Designated Contracting States:
**DE GB IT NL**

(56) References cited:
| EP-A-0 057 561 | US-A-2 532 788 |
| EP-A-0 071 131 | US-A-2 661 744 |
| DE-A-2 437 346 | US-A-3 593 718 |
| FR-A-1 047 798 | US-A-3 730 173 |
| FR-A-2 194 456 | US-A-3 773 051 |
| FR-A-2 257 312 | US-A-3 851 651 |
| FR-A-2 279 376 | US-A-4 009 721 |
| FR-A-2 305 168 | US-A-4 013 068 |
| FR-A-2 433 950 | US-A-4 308 870 |
| FR-A-2 493 154 | US-A-4 341 226 |
| GB-A-1 410 761 | US-A-4 467 807 |

(73) Proprietor: **MEDTRONIC, INC.**
**7000 Central Avenue, P.O. Box 1453**
**Minneapolis, MN 55440 (US)**

(72) Inventor: **MUGICA, Jacques**
**72, Avenue Henri Martin**
**F-75016 Paris (FR)**

(74) Representative: **Kopacz, William James**
**83, Avenue Foch**
**F-75116 Paris (FR)**

(56) References cited:
IEEE ENGINEERING IN MEDICINE AND
BIOLOGY, vol. 2, no. 2, June 1983, pages 27-31,
IEEE, New York, US; M.L. NOCHOMOVITZ:
"Electrical activation of respiration"

MEDICAL AND BIOLOGICAL ENGINEERING, vol.
14, no. 4, July 1976, pages 387-393, Stevenage,
GB; N. HOSHIMIYA et al.: "Basic studies on
electrophrenic respiration. Part 1 -
Electrophrenic respirator synchronised with
phrenic nerve impulses"

Courier Press, Leamington Spa, England.

**Description**

Background of the invention
1. Field of the invention

The instant invention relates to the stimulation of human body organs and relates more particularly to diaphragmic stimulation to effect respiration.

2. Description of the prior art

In a substantial number of instances, patients who undergo thoracic surgery may, for a varying period following the surgical procedure, suffer a temporary paralysis of their respiratory system. Such patients require ventilatory support during the post-operative recovery period. That support, which involves inducing artificial respiration, is typically effected by a mechanical respirator which applies a cyclic pressure to the pulmonary cavities. This is done directly by forcing air or oxygen into the lungs via the nasopharynx and the trachea, or indirectly by enclosing either the chest or the entire body with the exception of the head and neck and cyclicly pressurizing the enclosure so that the resulting chest movements stimulates natural breathing.

Similarly, persons who suffer traumatic nerve damage or contract degenerative neurological diseases, such as amyotrophic lateral sclerosis ("Lou Gehrig's" disease) have total respiratory paralysis which requires ventilatory support at all times. Again, in such situations, mechanical respirators of one form or another are employed in the hospital or home setting. The mechanical respirators prevent the patients from being ambulatory during the time that they are needed. For this reason and for the discomfort and other complications of the mechanical respirators, many have sought, over the last thirty-five years to develop a suitable, simple and effective replacement.

For those patients who have temporary or permanent respiratory paralysis, yet retain intact respiratory muscles, diaphragm and innervating nerve trunks from the cervical cord, it has been proposed to induce artificial respiration by electrically stimulating the phrenic nerve which, in turn, induces diaphragmatic contraction. In 1948, Sarnoff et al published the first paper in modern times on electrophrenic stimulation ("Electrophrenic Respiration", The American Journal of Physiology, Vol. 155, No. 1, October 1, 1948). Sarnoff et al proposed that a wire electrode be looped around the exposed phrenic nerve and exited through the skin for attachment to a laboratory stimulator. Stimulating pulses of suitable length and configuration would be conducted to the phrenic nerve which, in turn, would innervate the diaphragm (see also U.S. Patent 2,532,788).

Since 1948, many papers have been published on experiments conducted employing the concept of directly stimulating the phrenic nerve to induce respiration. Long-term phrenic nerve stimulation for diaphragm pacing was reported by Glenn et al in 1976 ("Long-Term Eventilator Support by Diaphragm Pacing in Quadriplegia" Ann. Surgery, 183, 566—576). Others have suggested that the complete stimulator and electrode system be completely implanted, either with permanent batteries or with a system for transcutaneously powering the implanted circuitry and electrode system (see Talonen et al, "Transcutaneous, Dual-Channel Phrenic Nerve Stimulator for Diaphragm Pacing", Med. & Biol. Eng. and Comput., 1983, Vol. 21, pgs. 21—30).

Further, in IEEE Engineering in Medicine and Biology, vol. 2, no. 2, June 1983, pages 27—31, there is suggested a manner of activating the diaphragm electrically. Although the electrodes in this publication are implanted in the diaphragm, the electrical impulses are used to stimulate the phrenic nerve with the magnitude of the contraction depending upon the proximity of the electrode to the main phrenic entering the diaphragm. Such a technique as it depends upon the phrenic nerve for activation of the muscle, depends upon the presence of a functioning phrenic nerve. Further, in chronic patients, the phrenic nerve will become fatigued because of the continuous electrical stimulation.

These percutaneous or implantable systems have not enjoyed a great deal of success, and mechanical respiration is the norm in medical practice today. In chronic implants, the phrenic nerve either becomes damaged or fatigued or otherwise fails to respond to the electrical stimulation after a period of time. In temporary situations, the complexity of the operation, including the employment of electrodes wrapped around the phrenic nerve, makes it easier to simply employ the mechanical respirator until the patient recovers.

Although others have conducted experiments of direct electrical stimulation of the diaphragm muscle itself (see Macoviak et al, "Electrical Conditioning of In-Situ Skeletel Muscle Replacement of Myocardium", Journal of Surgical Research, Vol. 32, pgs. 429—439, 1982), no one apparently has appreciated the possibility of employing the direct stimulation of the diaphragm itself to effect artificial respiration on a temporary basis.

Summary of the invention

This inventor has studied the possibility of stimulating the diaphragm in a manner which would provide the patient with efficient respiration assistance without discomfort (e.g., the "hiccup" effect) and which would employ a simple surgical procedure. Typically, the procedure would be conducted at the conclusion of open chest surgery to correct other problems.

According to the invention, there is provided an apparatus for diaphragmatic stimulation adapted to assist respiratory movement of a patient using stimulation electrodes and having appropriate means for applying electrical pulses to these electrodes. In particular, the invention is characterized in that it would include at least three stimulation electrodes which are adapted for implantation into or for affixation on the muscle tissues of at least one half portion of the diaphragm and wherein there is further included a means for applying a timed sequence of stimulation pulses to these electrodes with the individual electrodes receiving stimulation pulses at different times during each respiration cycle so as directly to stimulate the portions of the muscle of the diaphragm associated with each individual electrode in a time-staggered manner independently of the phrenic nerve to provoke a smooth and regular contraction of the diaphragm.

The invention also includes various additional features including the provision of various elements and units which are particularly adapted for use with the apparatus above discussed.

A number of leads, e.g. 6 active and up to 6 passive leads, each with one or more electrodes, may be passed through and along the diaphragm muscle to provide many points of stimulation. Stimuli may be applied sequentially to each active lead to provide rhythmic contraction of the diaphragm.

The time spacing between pulse trains as sequentially applied to the different electrodes, as well as the frequency or repetition sequence of the pulse trains, are variable in accordance with a predetermined program.

This repetition sequence is determined for each patient by testing the reactions of the patient to various stimulations. Once the best combination of values is determined, this combination is stored in a memory, and stimulation will then take place in accordance with these stored values.

An important advantage of the instant invention in regard to prior art respiration devices is as follows: in convention artificial respiration devices, air is brought into the lungs of the patient by pushing the diaphragm in a downward direction. This causes a problem as the externally directed force in the lungs of the patient also causes pressure to be applied against the auricle of the heart and thus interfers with blood circulation which in turn, retards the patient's rate of recovery. The diaphragm is displaced by electric means for the purpose of bringing air into the lungs. As there is no artifical forcing of air into the lungs, there is no pressure exerted against the heart and no obstacle to blood circulation.

Furthermore, this procedure avoids exposure of the phrenic nerve in the region of the neck and advantageously can be conducted following other chest surgery.

Following recovery by the patient, the transcutaneous leads can be withdrawn by traction similar to the manner in which a temporary heart pacing wire is retracted.

Brief description of the drawings

Other objects and many of the intended advantages of this invention will be readily appreciated as the same becomes better understood by reference to the following detailed description when considered in connection with the accompanying drawings in which like reference numerals designate like parts throughout the figures thereof and wherein:

Figure 1 is a schematic of the upper portion of the diaphragm in which are implanted stimulation electrodes according to the invention;

Figure 2 is a schematic view of a bipolar electrode system implanted in the diaphragm muscle;

Figure 3 is a schematic of a device for diaphragmic stimulation according to the invention;

Figure 4 is a diagram showing a one type of pulse which can be used for diaphragmic stimulation;

Figure 5 is another type of pulse which can be used for diaphragmic stimulation;

Figures 6 and 7 show two recordings of stimulation pulse trains;

Figure 8 shows examples of envelopes of stimulation signals;

Figure 9 shows a simplified flowchart of the method of triggering the delivery of stimulating pulses in the absence of spontaneous respiration;

Figure 10 illustrates how pulse trains are sequentially applied to the six outputs of the device shown in Figure 3 to effect a rhythmic contraction of the diaphragm;

Figure 11 shows a preferred unipolar, multi-electrode lead for implantation in the diaphragm for temporary stimulation; and

Figure 12 shows a cross-section view of a portion of the lead of Figure 11.

Detailed description of the preferred embodiments

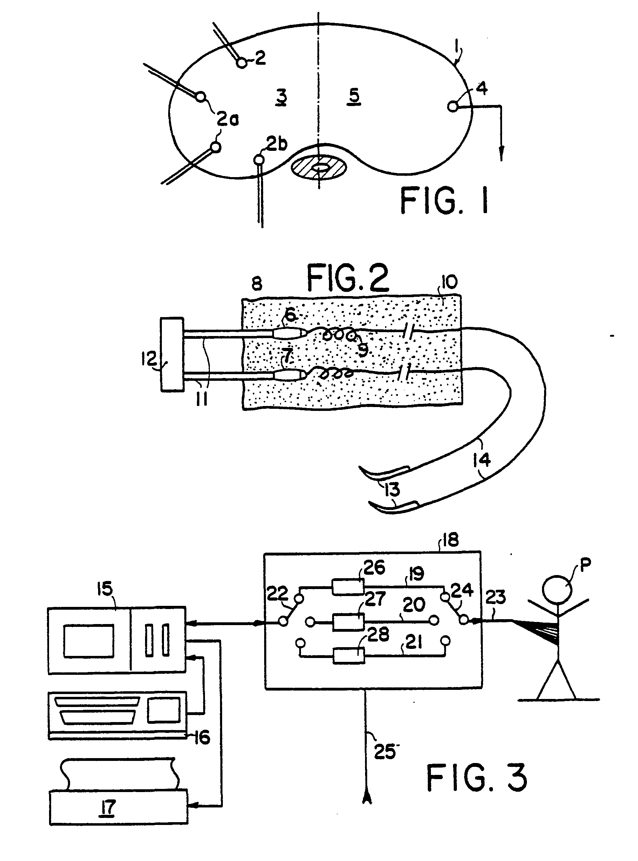

In Figure 1, there is shown schematically the muscle of diaphragm 1 with unipolar or bipolar electrodes 2 implanted in the muscle tissue at the external periphery of half of the diaphragm. There can be, for example, 20 such electrodes. Advantageously, sub-portions of these electrodes can be distributed along several lead wires, each having a common conductor.

The inventor has noted that the quality of contraction depends upon the emplacement selected for the electrodes in the diaphragm as well as the form of the stimulation pulse trains. It has been noted that the implantation of stimulation electrodes in only a single half of the diaphragm yields good results, and this also limits the amount of surgical intervention, which is in any case quite substantial.

In certain cases, as seen in Figure 1, a diaphragm movement sensor 4 is placed in the other half of the diaphragm 5. This is so it often occurs that one side of the diaphragm functions normally with the other half being incapacitated and requiring stimulation. In such cases, the sensor 4 detects the normal movement of the diaphragm and emits signals which are utilized (in the system of Figure 3) to trigger the stimulation of the other half of the diaphragm through electrodes contacting it so as to obtain a natural rhythm. The sensor 4 can be, for example, a pressure sensor, an impedance sensor, an EEG sensor or an activity sensor, and more than one can be provided. The activation of the stimulating electrodes will therefore be dependent upon the mechanical or electrical activity in the diaphragm portion 5 detected by sensor 4. Of course, sensors and electrodes may be placed on or in both portions 3 and 5 of the diaphragm.

Alternatively, a suitable blood gas sensor, e.g., a sensor for pH, pO2 or pCO2 could be employed in the blood stream to provide a control signal when oxygen tension falls below a preset limit to trigger operation of the respiration device of Figure 3.

Figure 2 shows schematically a bipolar single electrode lead of the type used in the invention. This lead is derived from the Medtronic temporary pacing lead model 6400 which is a temporary myocardial heart wire used for cardiac stimulation. In the Figure 2 embodiment, the lead consists of two electrodes 6 and 7 which receive positive and negative stimulation signals, or act as the active and passive stimulating electrodes.

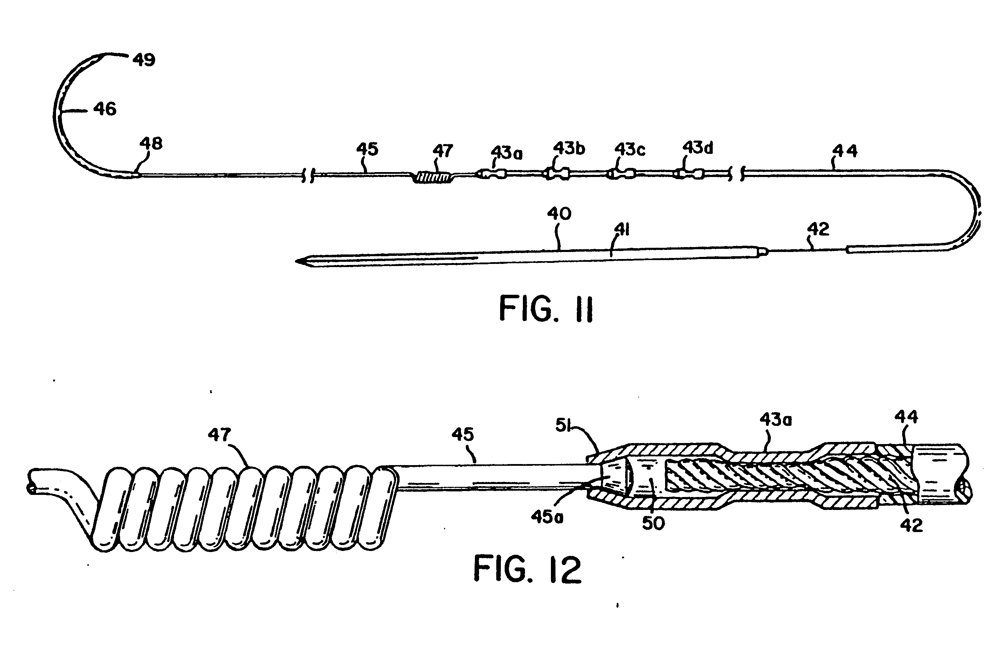

The bipolar lead includes ring electrodes 6 and 7 of biocompatible metal, such as for example, platinum, nonconductive filaments 14, each having a winding or coil 9 for fixing the electrodes into muscle 10, and lead bodies 11 extending to connector 12 for attachment to an excitation signal generator which will be discussed in connection with Figure 3. Each lead body and electrode is drawn into the muscle 10 by perforating the muscle with its respective needle 13 and drawing the filament 14 and coil 9 into the muscle. The filament 14 is severed leaving the coil 9 in place to retain the electrode 6 or 7 in place. Other types of leads, for example, one or more unipolar leads having several electrodes of the type shown in Figures 11 and 12 can also be used.

The electrodes can be implanted by open thoracic or open abdomenal surgical intervention. Implantation can also be effected by endoscopy, by pleuroscopy or peritonioscopy. The lead may later be retracted to pull the electrodes and coils out of the diaphragm and out of the patient's body through the skin incision.

Figure 1 represents the electrodes which are implanted in the diaphragm; however, these electrodes might also be affixed to a net or a plastic film element or similar, and this net or plastic film element might then be affixed to the diaphragm, the electrodes being placed in physical contact with the upper or lower portions of the diaphragm. In this manner, electric energy can be delivered to the diaphragm without a physical implantation of the electrodes within the diaphragmatic muscle. However, such an arrangement would not be retractable through the skin in the fashion that the temporary lead wire described herein may be used.

Figure 3 shows the overall arrangement of the diaphragmatic stimulation device according to the invention. The stimulation apparatus shown in this figure includes a user interface 15 to which are connected a keyboard 16 and a printer 17. The interface 15 can be a micro-computer ELTEC system 7000 with a 6809 processor, a 64K memory and two 320K disc readers which effect the following functions:

Modification of stimulation parameters such as pulse amplitude, frequency and width and the envelope of the stimulation signals as well as the time interval between different pulse trains as well as the electrode choice;

Calculation and display of the stimulation data;

Data control, storing of the stimulation program, control of the printer, programming the stimulator.

The interface 15 is connected to a stimulator 18 through several, e.g. six, for example, independent floating unipolar or bipolar capacitive coupling outputs of the constant current or constant voltage type (or any other source of energy) connected to electrodes such as electrodes 2 of Figure 1 implanted in patient P.

The stimulator 18 includes a permanent stimulation path 19, a temporary stimulation path 20 and an emergency stimulation path 21; these three paths are connected to interface 15 by means of a commutator 22 and are connected to patient P by means of cable 23 and commutator 24. These commutators provide for stimulation independent of the interface 15 after programming. The stimulator 18 includes in addition an input 25 for remote control.

The control signal from the sensor 4 of Figure 1 or other sensors as described above may be applied to the interface 15 which responds through suitable software to interrupt the delivery of stimulating or excitation pulse trains when the patient experiences spontaneous respiration of the blood gas levels are within normal range. In Figure 3, it is presumed that the control signal from sensor 4 is delivered in time sequence from patient P to interface 15 via cable 23 and stimulator 18. The signal from sensor 4 may instead be separately input directly into interface 15. Similarly signals developed by blood gas sensors or internal impedance plethysomograph signals may be developed from electrodes spaced apart on the diaphragm or in the chest cavity may be applied to interface 15. Alternatively an external thoracic belt with spaced electrodes can be used temporarily to develop an impedance plethysmograph control signal directly inputted into interface 15.

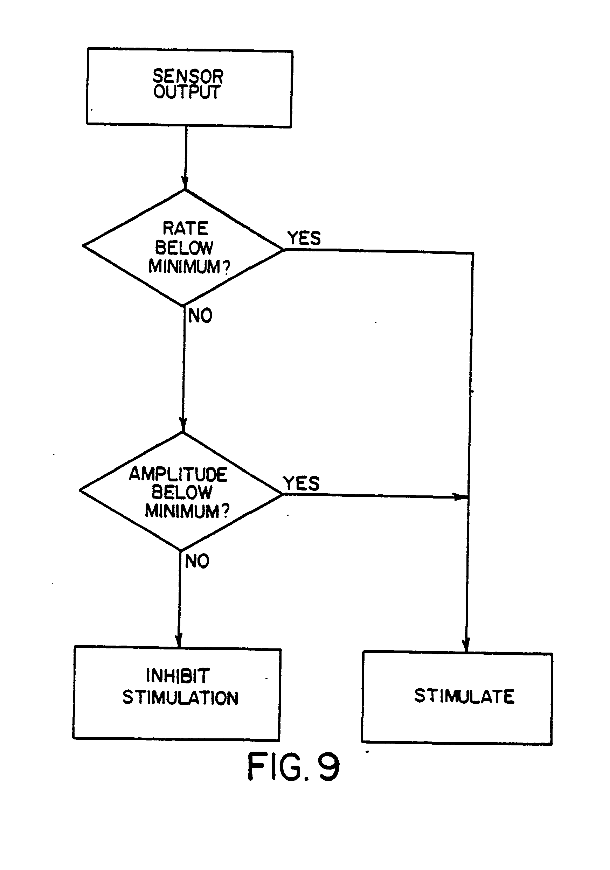

Figure 9 illustrates how the sensor output is utilized to provide "inhibit" or "stimulate" control signals through commutator 22 to stimulator 18. In this example, the sensor develops an electrical signal representative of diaphragmic contraction, the signal having an amplitude representative of the strength of contraction. The interval between successive contractions is inverted to provide a contraction rate.

In Figure 9, the sensor output (rate and amplitude) is first compared to a minimum rate; if the rate is below the minimum, a "stimulate" command is given to pulse generator 18. If the rate is above the minimum, then the amplitude is compared to a minimum amplitude. If the amplitude is below the minimum, then a "stimulate" command is given even though the rate, or interval between successive respirations, is acceptable. If the amplitude is above the minimum, then the "inhibit" command is given to the pulse generator 18. Thus, to inhibit, both rate and amplitude must be within normal ranges; to stimulate either rate or amplitude need be below normal.

Although, in the embodiment described, the control means for stimulation are external to the body, it is clear that the stimulator can also be implanted in the body and programmed from the exterior. Such implanted stimulators utilizing external programming means are widely used today in cardiac stimulation, with the program stored in the implanted stimulator being controlled and modified remotely from outside of the body by electromagnetic means, for example. These same principles can be applied to the diaphragmatic stimulator according to the instant invention.

The utilization of pulse trains instead of isolated pulses causes a diaphragm contraction which is regular and permits the generation of the necessary energy needed to overcome the elastic resistance of the tissues, the resistance of the displacement of the air and to assure the displacement of the abdominal mass.

The phenomena of muscular fatigue causes a reduction in the contraction response to an electric stimulation occasioned by irreversible electro-chemical reactions at the muscle nerve electrode interface. In order to reduce this phenomena, the electric pulses must satisfy the following conditions:

Equilibrium in the electric charge within a pulse interval. The negative charge of the pulse which causes the muscle contraction should be balanced by the same charge quantity during the positive phase. Of course, the lead pulse which causes the contraction can be positive, and the following pulse can be negative;

The intensity of the current during the time of the positive and negative pulse phases must be the same.

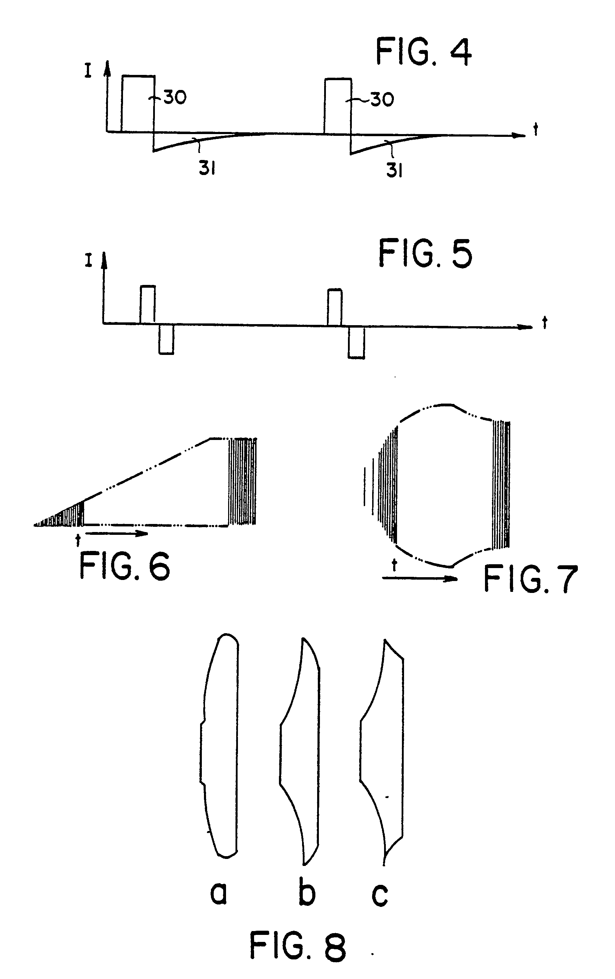

A pulse which would satisfy the first condition is seen in Figure 4 in which the area of the positive rectangular pulses 30 and the area of the negative triangular pulses 31 are equal. The second condition would be satisfied by the pulses shown in Figure 5. A pulse which satisfies both conditions will provide better resistance to fatigue than a pulse which satisfies only the first condition.

It has been noted that the best results are probably obtained when the form of the positive and negative current pulse waves are identical, such as seen in Figure 5. The response of the tissues to identical pulses of opposite polarity will be improved if a pulse is displaced in time with respect to the other pulse, and there will be no unbalanced tissue reaction which would occur if the current pulses were of different forms as seen in Figure 4. The positive and negative pulses of Figure 5 need not be adjacent to one another as seen in this figure but rather can be displaced with a time interval between the positive and negative pulses.

There will now be described the trains of pulses utilized in diaphragmatic stimulation. A train of pulses can be characterized by three parameters:

the envelope of the amplitude of the pulses;

the envelope of the width of the pulses;

the envelope of the frequency of the pulses.

These three envelopes can include an increasing phase, a constant phase and a decreasing phase which can be linear or curved. One can vary the form of the envelopes and adjust them in a way to obtain the desired contraction of the diaphragm muscles.

In Figures 6 and 7, there are shown two representations of actual recordings of pulse trains. Figure 6 shows a monophase train of pulses having a linear amplitude envelope and a constant envelope as to pulse width and frequency.

Figure 7 shows a biphasic train of pulses having a curved amplitude envelope, a curved frequency envelope and a constant pulse width envelope. The table which follows show, as an example, the most significant parameters and their particular range adapted to this particular application

| Variation | Range |
| --- | --- |
| Output | Active-inactive |
| Repetition frequency | 0.1—200 bursts per minute |
| Pulse mode | Mono or Biphasic or Alternative |
| Pulse polarity | Positive or Negative |
| Pulse delay | 0.0005—999 ms |
| Envelope mode | Linear or exponential or any other curve |
| Pulse amplitude | 200—20 mA |
| Pulse width | 0.005—50 ms |
| Pulse frequency | 0.1—200 Hz (preferably 4—50 Hz) |

The interval between successive biphasic pulses may preferably be adjustable between 0.5 and 5 ms. The characteristics of the envelopes, such as the repetition frequency, the stimulation fraction in the respiration cycle, the amplitude and the form of the envelope can be modified within the substantial range set forth above. Pulse generator 18 and/or the interface 15 may be designed with a circuit and/or software, respectively, to prevent the misprogramming through keyboard 16 or component failure from causing excessively high stimulating parameters, e.g. excessive rates and amplitudes of the excitation pulses.

Examples of variations which engender corresponding variations in the stimulation energy of the associated electrode are represented in Figure 8 which shows exponential envelopes of biphasic pulses. It will be recognized that the response of a diaphragmic region will be different according to whether it receives pulse trains having envelopes a, b or c.

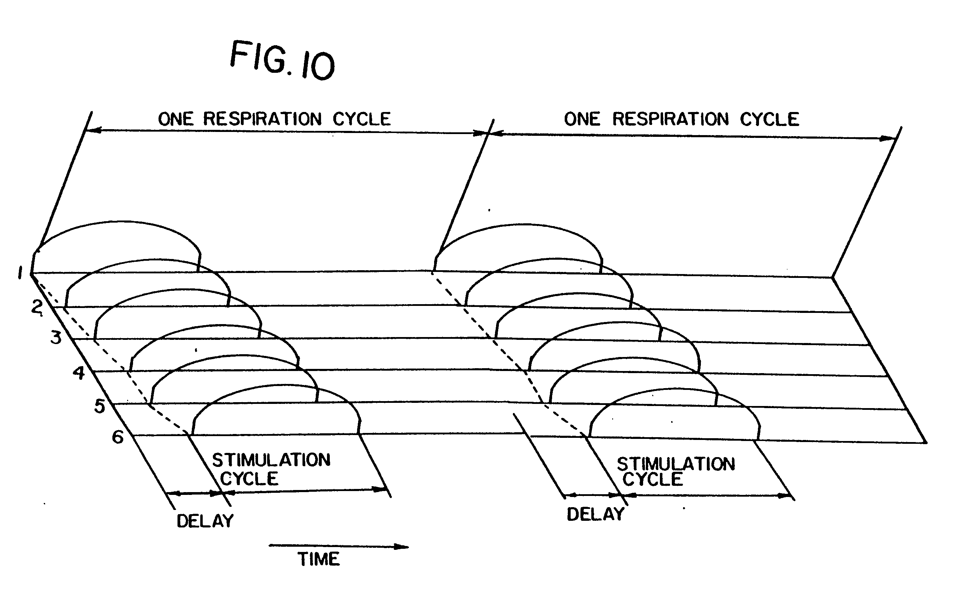

The device which has been described produces therefore a modulated stimulation with the possibility of modification of the parameters of the pulse trains and of each pulse within the trains. Figure 10 illustrates how individual pulse trains are sequentially applied to the six outputs of the device shown in Figure 3 to effect a rhythmic contraction of the diaphragm.

In determining the waveforms of the pulses to be applied to the electrodes of Figure 1, it is important to note that a particular burst of pulses will be applied, for example, to a particular electrode. Another different burst of pulses will then be applied to another electrode with this latter burst being delayed somewhat with respect to the first burst by, for example, 10 to 100 ms. A third burst, also delayed, will then be applied to a third electrode and so forth. These delays are shown in Figure 10 for all six output pulse trains, and they may vary from 0 to 20% of each stimulation cycle. The stimulation cycle may itself extend over 6 to 60% of the respiration cycle. In this way, electric energy will be applied to the diaphragm in a time delayed manner which will cause a contraction which is more regular and avoid spasmatic contractions.

Also, in regard to each particular pulse burst, applied to any particular electrode, the waveforms of the pulses of the bursts can vary. For example, the first pulse can be of a weak amplitude and/or duration with the following pulses increasing in amplitude and/or duration. Similarly, the frequency of the pulses in a particular burst can vary within the burst. All of this can contribute to obtaining a respiration which is regular and smooth.

The total group of pulses delayed in time applied to all of the electrodes is of course repeated for each successive respiration as illustrated in Figure 10. In determining the appropriate characteristics of the bursts, the response of the patient to various characteristics will be observed, and the particular sequence which is the best suited to the particular patient will be noted and placed in memory. It is not otherwise possible to set forth a particular set of characteristics for the bursts which will function best for all patients or for all electrode emplacement arrangements.

The numerous possibilities of programming the device allows one to vary, in a continuous and independent manner, the amplitude, width and frequency of the pulses within the envelope. The envelopes effect a global stimulation in regard to the part of the diaphragm associated with the electrode pair. The start of each stimulation train can be programmed as to its arrival time at the stimulation point.

The operation of the interface 15

The programmer provides the means for entering and modifying the parameters and the stimulation control signals which appear on the screen of the interface device 15. In response to an appropriate instruction, the stimulation characteristics, namely the width, duration and pulse frequency, will be calculated from a group of parameters and will be applied to one of the three independent registers 26, 27, 28 of stimulator 18.

Once the three registers are loaded, stimulator 18 will commence stimulation by reading the register 26, 27 or 28 which is selected and generating electric pulses conforming with the contents of the register after execution of an appropriate instruction. Once the stimulator is functioning the programmer can modify parameters and can execute other steps without affecting the stimulation function. The operations seen on the screen can be memorized on a disk, printed or programmed.

An example of the available program controls is given below.

          1—"stimulation request"—permanent "return"
                                 —temporary
                                 —emergency
                                 —name

The stimulation processing in the indicated register or the processing memorized on a disk under the name which is printed on keyboared 16 is transferred to the programmer memory and assigned to the interface monitor.

Then the following instructions are given:

          2—"programs"—permanent "return"
                            —temporary
                            —emergency

The operations on the screen are transferred to the selected stimulation register. Each register can be selected independently of the state of the stimulator, whether it is in operation or not.

If the operation is changed in the register in which the stimulator is functioning, the stimulator restarts automatically at the end of the last stimulation cycle after the end of the data transfer.

> 3—"proceed"—permanent "return"
> —temporary
> —emergency

The stimulator starts the execution of the treatment in the indicated register. If no register is indicated, the preceding register or the one currently being used in selected.

> 4—"stop" "return"

The stimulator stops at the end of the stimulation cycle in progress.

In order to test and verify the operation of the device, clinical experiments were conducted on 9 patients aged from 14 to 75 years. The choice criteria of the patients was not in regard to diaphragmic stimulation factors per se, but rather in regard to the possibility of implanting the electrodes without encountering the slightest risk to the patients. Of the 9 patients, 4 retained their electrodes for 3 to 7 days. The fundamental result was that it was determined with certainty that there could be produced a diaphragmatic stimulation of a good quality in the sense of a muscular stimulation which restores normal respiratory movement. Although the types of diaphragms were quite different, a respiratory movement was always produceable after the patient had been appropriately tested.

The findings were the following:

The quality of the contraction is variable as a function of diaphragm characteristics (a macroscopic determination by the surgeon);

The stimulation parameters are variable as function of diaphragm characteristics and the electrode implantation technique.

The best contraction observed was with stimulation impulses which were biphasic, alternating, and with the optimum delay between the pulses within a train being on the order of 2 milliseconds.

After various attempts, all types of contractions were obtained: tremulations, partial contractions in the zone peripheral to each electrode, and hiccups. When the parameters of the envelope of stimulation were correctly determined, the diaphragmic contraction obtained was progressive, global, homogenous and without the fatigue phenomena.

Diaphragmic stimulation was attempted on 2 patients upon awakening from the anesthesia administered during surgery. There was noted under stimulation an increase of 2.41/mn on the spirometer compared with spontaneous respiration.

So as to be able properly to interpret the experimentation, in particular because of the painful post-operative period, stimulations were again effected some time after the intervention. In regard to the same patient who had been tested upon awakening, each stimulation was followed by a better quality expiration and an amelioration of the useful yield. In regard to the same patient, the initial stimulation with the same threshold value as per-operatory was painful, but if the threshold values were increased progressively, but rather rapidly, the procedure was no longer painful.

A second patient did not experience any painful phenomena during a stimulation which lasted 20 minutes. During this stimulation, expirations were noted having amplitudes which were greater following each stimulation, interspersed with spontaneous respiratory movements which were less pronounced.

It is noted that better results can be obtained by reducing, or otherwise modifying, the cyclical stimulation relation. The optimum procedure is not necessarily to stimulate the diaphragm for each inhalation but rather, for some patients, the best results can be obtained by one or more artificial stimulations which can be followed by one or more natural respiratory movements, with another stimulation then being applied and so forth. Such sequences can be programmed in advance with the apparatus according to the invention.

The retractable diaphragm wire

A preferred form of diaphragm wire that carries several spaced electrodes and may be retracted through the skin is shown in Figures 11 and 12. This wire or lead is similar to the temporary heart pacing lead shown and described in U.S. Patent 4,341,226, and its insertion in the diaphragm is similar to the manner in which the referenced heart lead is inserted into the heart wall.

Referring to Figure 11, the proximal end of the lead contains a metallic, electrically conductive needle 40 which is used as a connector pin to couple the lead to the output terminals 23 of the pulse generator 18. The needle 40 may have a weakened section 41 which may be severed after implant so that only the remaining section of the needle 40 is so used. Needle 40 is attached to the flexible conductor 42 which runs the length of the temporary pacing lead and is attached to electrodes 43a, 43b, 43c and 43d, etc. Insulating sheath 44 is of a material substantially inert to body fluids such as polyethylene. Sheath 44 also insulates the conductor 42 extending between the electrodes 43a—43d. A length of surgical thread 45 is attached between the electrode 43a and curved needle 46. Surgical thread 45 has an outside diameter of about 0.35 mm and is commonly available type such as polypropylene. Helix 47 is molded into the surgical thread by

7

applying hot air to the coil surgical thread. Curved needle 46 is a standard surgical needle, the surgical thread 45 is attached to the curve needle 46 by a crimp at 48. Surgical needle 46 terminates in a hand-honed needle point 49.

Figure 12 is a sectional view of electrode 43a. The total length of electrode 43a is about 3 mm. The outside diameter of sheath 44 is approximately 0.7 mm. Sheath 44 extends between the proximal end of electrode 43a and the distal end of electrode 43b. Conductor 42 passes through the lumens of sheath 44 and electrodes 43a—43d. In each case, the electrode is crimped down around the conductor 42 in the manner depicted in Figure 12. The proximal end of surgical thread 45 is enlarged to produce fastener 45a. This may be accomplished by heating the proximal end of surgical thread 45 and applying it through force in the distal direction, thus flattening the end.

Fastener 45a is inserted into chamber 50 which lies within electrode 43a between the distal end of conductor 42 and the distal end of electrode 43a. The distal end 51 of electrode 43a is swaged, producing the conical frustrum shape which securely holds fastener 45a.

The remaining electrodes 43b—43d have a shape similar to the electrode 43a except for the conical frustrum end 51. The proximal and distal ends of these electrodes butt up against the insulating sheath 44 in the manner shown at the proximal end of electrode 43a in Figure 12.

The temporary diaphragm wires of the type shown in Figures 11 and 12 may have any number of electrodes 43 as may be found desirable, and the spacing between electrodes may be varied as considered desirable. In each case, the electrodes 43 are electrically connected in series with the conductor 42 so that electrical impulses applied through the wire simultaneously stimulate a number of points in and around the diaphragm that the electrodes are threaded through.

In use, one or more of the lead wires may be surgically attached to the diaphragm and coupled to one of the six or so outputs of the pulse generator 18. The method of implantation is described more completely in the aforementioned U.S. Patent 4,341,226. As stated earlier, the helical coil 47 retains the electrodes in the diaphragm after the surgical thread 18 has been severed. The length of the conductor extending between the final electrode 43d and the connector pin provided by the needle 40 is exited through the skin and coupled to the output of the external pulse generator. The wire is retracted by pulling on the exteriorized portion of the lead and straightening the coil 47 which allows the entire lead to be retracted from the body. For bipolar stimulation, one or more heart wires may be used as the active and indifferent electrode sets.

## Claims

1. An apparatus for diaphragmatic stimulation adapted to assist respiratory movements of a patient using stimulation electrodes and means for applying stimulation pulses to said electrodes, the apparatus characterized in that it includes:

at least three stimulation electrodes (2, 2a, 2b) for implantation into, or for affixation on, the muscle tissues of at least one-half portion of the diaphragm (1, 3 of Figure 1), and

means for applying (15, 18 of Figure 3) to the said electrodes a timed sequence of stimulation pulses with the individual electrodes receiving stimulation pulses at different times during each respiration cycle so as to directly to stimulate the portions of muscle of the diaphragm associated with each individual electrode in a time staggered manner independently of the phrenic nerve to provoke a smooth and regular contraction of the diaphragm.

2. The apparatus of Claim 1 wherein the means for applying (15, 18) includes stimulating means (18) for applying to the electrodes, in accordance with a predetermined sequence, particular stimulation pulses during each respiration cycle, so as directly to stimulate the portions of muscle of the diaphragm associated with each individual electrode in a time staggered manner, and interface means (15) for providing command signals for the stimulation pulses.

3. The apparatus according to Claim 2 wherein the stimulating means (18) includes three paths for permanent, temporary and emergency stimulation, (19, 20, 21) and means for connecting these paths (22, 24) to said interface means and to a lead means (11).

4. The apparatus according to Claims 2 or 3 wherein the stimulation electrodes (2) are bipolar and are each formed of two electrode elements of biocompatible metal having means for fixation into the muscular tissues and having curved needles (13) and transcutaneous needles connected to a connector (14) for connection with the stimulator means.

5. The apparatus according to Claims, 2, 3, or 4 further comprising:

a) sensor means (4) for detecting the activity of at least one-half portion (4) of the diaphragm; and

b) further means for connecting said sensor means to the stimulating means for triggering the stimulation pulses in one or both halves (3, 5) of the diaphragm (1) following the output signal of the sensor means (4) detecting natural diaphragm activity in at least one-half of the diaphragm to provide synchronous contraction of both halves of the diaphragm.

6. The apparatus according to Claim 5 further comprising further sensor means (4) for detecting the activity of the other half portion of the diaphragm.

7. The apparatus according to any preceding claim wherein the lead means (11) is adapted to be placed

EP 0 190 195 B1

against the diaphragm (1) with the stimulating electrodes (2) in contact with the external surface of the diaphragm.

8. The apparatus according to any of the preceding claims wherein:

a) the lead means (11) comprises a plurality of spaced apart electrodes (2) in contact with diaphragm tissue; and

b) the stimulating means (18) includes means for applying bursts of stimulation pulses to said electrodes with a delay between bursts (Figure 10) applied to different electrodes so as to provide a rhythmic contraction of the diaphragm.

9. The apparatus according to Claim 8 wherein the bursts consist of stimulation pulses having a variable frequency and/or amplitude and/or duration.

10. The apparatus according to Claim 8 or 9 wherein the stimulation pulses have positive and negative current pulse waveforms which are substantially identical.

11. The apparatus according to any preceding claim wherein the stimulating pulses applied to the stimulation electrodes according to a predetermined sequence are constituted by monophase or biphase pulse trains, or alternating monophase—biphase pulse trains, modulated as to width and/or amplitude and/or frequency, with a delay being provided between the pulse trains arriving at different electrodes, the respiration frequency of the pulse trains determining the respiration cycle.

12. The apparatus according to Claim 11 wherein the envelopes of said pulse train include increasing and decreasing portions which are linear or curvilinear.

13. The apparatus according to any preceding claim further comprising:

a) sensor means attached to or implanted in the body of the patient for detecting a physiological parameter associated with respiration activity and developing a respiration amplitude control signal having a value reflecting the strength of contraction of the diaphragm; and

b) means for applying the stimulation pulses to the stimulation electrodes when the value of said respiration amplitude control signal indicates the need for respiratory assistance.

14. The apparatus according to any of Claims 2 to 12 further comprising:

a) sensor means adapted to be attached or implanted on or in the body of the patient for detecting a physiological parameter associated with respiration activity and developing a respiration rate control signal reflecting the interval between successive spontaneous respirations of the patient; and

b) means for applying said stimulation pulses to said stimulation electrodes when the respiration rate control signal reflects an interval greater than a predetermined reference interval.

15. An apparatus according to Claim 14 further comprising:

a) means responsive to said sensor means for developing a respiration amplitude control signal having a value reflecting the strength of contraction of the diaphragm; and

b) means for applying the stimulation pulses to the stimulation electrodes when the value of said respiration amplitude control signal indicates the need for respiratory assistance.

16. The apparatus according to Claim 13, 14, or 15 wherein said sensor means (4) detects the EEG of the diaphragm or derives an impedance plethysmograph signal from spaced apart electrodes implanted on or adjacent to the diaphragm or worn on the patient's thorax and/or comprises a motion sensor implanted adjacent to or on the diaphragm (1) for developing a signal in response to movement of the diaphragm.

17. The apparatus according to any preceding claim further comprising:

a) sensor means adapted to be implanted in the bloodstream of the patient for detecting a physiological blood parameter associated with respiration and developing a control signal in response thereto; and

b) means for applying the stimulation pulses to said stimulation electrodes when the control signal reflects a blood parameter indicative of the need for respiration.

18. A device according to Claim 17 wherein the sensor detects blood pH, pO2 and/or pCO2.

## Patentansprüche

1. Vorrichtung für die Diaphragmastimulation zur Unterstützung der Atembewegungen eines Patienten unter Verwendung von Stimulationselektroden und Mitteln zum Anlegen von Stimulationsimpulsen an diese Elektroden, gekennzeichnet durch:

mindestens drei Stimulationselektroden (2, 2a, 2b) zur Implantation in oder zur Befestigung an Muskelgeweben mindestens der einen Hälfte des Diaphragmas (1, 3 in Fig. 1), und

eine Applikatoranordnung (15, 18 in Fig. 3) zum Anlegen einer zeitlichen Folge von Stimulationsimpulsen an die Elektroden, wobei den einzelnen Elektroden Stimulationsimpulse zu unterschiedlichen Zeiten während jedes Atemzyklus zugehen, um unmittelbar die jeder einzelnen Elektrode zugeordneten Muskelteile des Diaphragmas in zeitversetzter Weise unabhängig von dem Phrenikus zu stimulieren und dadurch eine gleichmäßige und regelmäßige Kontraktion des Diaphragmas zu provozieren.

2. Vorrichtung nach Anspruch 1, wobei die Applikatoranordnung (15, 18) eine Stimulationseinrichtung (18) zum Anlegen von einzelnen Stimulationsimpulsen an die Elektroden entsprechend einer vorbestimmten Abfolge während jedes Atemzyklus zwecks unmittelbarer Stimulation der jeder einzelnen Elektrode zugeordneten Muskelteile des Diaphragmas in zeiversetzter Weise, sowie eine

9

Schnittstellenanordnung (15) zum Anliefern von Befehlssignalen für die Stimulationsimpulse aufweist.

3. Vorrichtung nach Anspruch 2, wobei die Stimulationseinrichtung (18) drei Wege für permanente Stimulation temporäre Stimulation und Notstimulation (19, 20, 21) sowie Mittel (22, 24) zum Anschließen dieser Wege an die Schnittstellenanordnung und an eine Leitungsanordnung (11) aufweist.

4. Vorrichtung nach Anspruch 2 oder 3, wobei die Stimulationselektroden (2) bipolar und jeweils aus zwei Elektrodenelementen aus biokompatiblem Metall gebildet sind, sowie Mittel zur Fixierung in den Muskelgeweben und gekrümmte Nadeln (13) sowie transkutane Nadeln aufweisen, die mit einem Anschlüß (14) zum Verbinden mit der Stimulationseinrichtung verbunden sind.

5. Vorrichtung nach Anspruch 2, 3 oder 4 ferner versehen mit:

(a) einer Sensoranordnung (4) zum Efassen der Aktivität mindestens einer Hälfte (4) des Diaphragmas; und

(b) weiteren Mitteln zum Verbinden der Sensoranordnung mit der Stimulationseinrichtung zum Auslösen der Stimulationsimpulse in einer oder beiden Hälften (3, 5) des Diaphragmas (1) im Anschluß an das Ausgangssignal der natürliche Diaphragmaaktivität in mindestens einer Hälfte des Diaphragmas erfassenden Sensoranordnung (4) um für synchrone Kontraktion beider Hälften des Diaphragmas zu sorgen.

6. Vorrichtung nach Anspruch 5, ferner versehen mit einer weiteren Sensoranordnung (4) zum Erfassen der Aktivität der anderen Hälfte des Diaphragmas.

7. Vorrichtung nach einem der vorhergehenden Ansprüche, wobei die Leitungsanordnung (11) an das Diaphragma (1) derart anlegbar ist, daß die Stimulationselektroden (2) mit der Außenfläche des Diaphragmas in Kontakt stehen.

8. Vorrichtung nach einem der vorhergehenden Ansprüche, wobei:

(a) die Leitungsanordnung (11) eine Mehrzahl von in Abstand voneinander liegenden Elektroden (2) aufweist, die mit Diaphragmagewebe in Kontakt stehen; und

(b) die Stimulationseinrichtung (18) Mittel zum Anlegen von Paketen von Stimulationsimpulsen an die Elektroden aufweist, wobei zwischen den an unterschiedliche Elektroden angelegten Impulspaketen (Fig. 10) eine Verzögerung vorgesehen ist, um für eine rhythmische Kontraktion des Diaphragmas zu sorgen.

9. Vorrichtung nach Anspruch 8, wobei die Pakete aus Stimulationsimpulsen mit variabler Frequenz und/oder Amplitude und/oder Dauer bestehen.

10. Vorrichtung nach Anspruch 8 oder 9, wobei die Stimulationsimpulse positive und negative Stromimpulswellenformen haben, die im wesentlichen identisch sind.

11. Vorrichtung nach einem der vorhergehenden Ansprüche, wobei die entsprechend einer vorbestimmten Folge an die Stimulationselektroden angelegten Stimulationsimpulse von einphasigen oder zweiphasigen Impulszügen oder von alternierend einphasigen-zweiphasigen Impulszügen gebildet sind, die hinsichtlich ihrer Breite und/oder Amplitude und/oder Frequenz moduliert sind, wobei zwischen den an unterschiedlichen Elektroden ankommenden Impulszügen eine Verzögerung vorgesehen ist und wobei die Folgefrequenz der Impulszüge den Atemzyklus bestimmt.

12. Vorrichtung nach Anspruch 11, wobei die Einhüllenden des Impulszuges ansteigende und abfallende Teile aufweisen, die linear oder gekrümmt sind.

13. Vorrichtung nach einem der vorhergehenden Ansprüche ferner versehen mit:

(a) einer am Körper des Patienten angebrachten oder dort implantierten Sensoranordnung zum Ermitteln eines der Atemaktivität zugeordneten physiologischen Parameters und zur Bildung eines Respirationsamplituden-Steuersignals, das einen die Stärke der Kontraktion des Diaphragmas widerspiegelnden Wert aufweist; und

(b) einer Anordnung zum Anlegen der Stimulationsimpulse an die Stimulationselektroden, wenn der Wert des Respirationsamplituden-Steuersignals den Bedarf an einer Unterstützung der Atmung erkennen läßt.

14. Vorrichtung nach einem der Ansprüche 2 bis 12 ferner versehen mit:

(a) einer am Körper des Patienten anzubringenden oder dort zu implantierenden Sensoranordnung zum Ermitteln eines der Atemaktivität zugeordneten physiologischen Parameters und zur Bildung eines Respirationsfrequenz-Steuersignals, welches das Intervall zwischen aufeinanderfolgenden spontanen Atemvorgängen des Patienten widerspiegelt; und

(b) einer Anordnung zum Anlegen der Stimulationsimpulse an die Stimulationselektroden, wenn das Respirationsfrequenz-Steuersignal ein Intervall erkennen läßt das größer als ein vorbestimmtes Bezugsintervalls ist.

15. Vorrichtung nach Anspruch 14, ferner versehen mit:

(a) einer auf die Sensoranordnung ansprechenden Einrichtung zur Bildung eines Respirationsamplituden-Steuersignals, das einen die Stärke der Kontraktion des Diaphragmas widerspiegelnden Wert aufweist; und

(b) einer Anordnung zum Anlegen der Stimulationsimpulse an die Stimulationselektroden, wenn der Wert des Respirationsamplituden-Steuersignals den Bedarf an einer Unterstützung der Atmung erkennen läßt.

16. Vorrichtung nach Anspruch 13, 14 oder 15, wobei die Sensoranordnung (4) das EEG des Diaphragmas erfaßt oder ein Impedanz-Plethysmographen-Signal von in Abstand voneinander liegender Elektroden ableitet, die an oder benachbart dem Diaphragma implantiert sind oder an dem Thorax des Patienten getragen werden und/oder einen Bewegungssensor aufweist, der benchbart dem oder an dem Diaphragma (1) implantiert ist, um ein Signal in Abhängigkeit von der Bewegung des Diaphragmas zu bilden.

17. Vorrichtung nach einem der vorhergehenden Ansprüche ferner versehen mit:

(a) einer in den Blutstrom des Patienten implantierbaren Sensoranordnung zum Erfassen eines der Atmung zugeordneten physiologischen Blutparameters und zur Bildung eines Steuersignals in Abhängigkeit davon; und

(b) einer Anordnung zum Anlegen der Stimulationsimpulse an die Stimulationselektroden, wenn das Steuersignal einen Blutparameter widerspiegelt, der einen Bedarf an einer Unterstützung der Atmung erkennen läßt.

18. Vorrichtung nach Anspruch 17, wobei der Sensor den pH-Wert, den pO2-Wert und/oder den pCO2-Wert des Blutes erfaßt.

## Revendications

1. Dispositif de simulation diaphragmatique adapté pour assister les mouvements respiratoires d'un patient moyennent l'utilisation d'électrodes de stimulation et de moyens pour appliquer des impulsions de stimulation auxdites électrodes, le dispositif étant caractérisé en ce qu'il inclut:

au moins trois électrodes de stimulation (2, 2a, 2b) destinées à être implantées dans ou fixées sur les tissues musculaires d'au moins une moitié du diaphragme (1, 3 sur la figure 1), et

des moyens (15, 18 sur la figure 3) pour appliquer auxdites électrodes une séquence cadencée d'impulsions de stimulation, les électrodes individuelles recevant des impulsions de stimulation à des instants différents pendant chaque cycle respiratoire de manière à stimuler directement les parties du muscle du diaphragme associées à chaque électrode individuelle, d'une manière échelonnée dans le temps, indépendamment du nerf phrénique pour provoquer une contraction dource et régulière du diaphragme.

2. Dispositif selon la revendication 1, dans lequel les moyens d'application (15, 18) incluent des moyens de stimulation (18) servant à appliquer aux électrodes, conformément à une séquence prédéterminée, des impulsions particulières de stimulation pendant chaque cycle respiratoire, afin de stimuler directement, d'une manière échelonnée dans le temps, les parties du muscle du diaphragme associé à chaque électrode individuelle, et des moyens d'interface (15) servant à envoyer des signaux de commande pour les impulsions de stimulation.

3. Dispositif selon la revendication 2, dans lequel les moyens de stimulation (18) incluent trois voies (19, 20, 21) permettant une stimulation permanente, une stimulation temporelle et une stimulation d'urgence, des moyens (22, 24) pour raccorder ces voies auxdits moyens d'interface et à des moyens formant conducteurs (11).

4. Dispositif selon la revendication 2 ou 3, dans lequel les électrodes de stimulation (2) sont bipolaires et sont constituées chacune de deux éléments d'électrode formés d'un métal biocompatible comportant des moyens pour leur fixation dans les tissus musculaires et possédant des aiguilles courbes (13), et des aiguilles transcutanées raccordées à un connecteur (14) pour le raccordement aux moyens de stimulation.

5. Dispositif selon les revendications 2, 3 ou 4, comprenant en outre:

a) des moyens formant capteur (4) pour détecter l'activité d'au moins une moitiée (4) du diaphragme; et

b) d'autres moyens pour raccorder lesdits moyens formant capteur aux moyens de stimulation pour déclencher les impulsions de stimulation dans une moitié ou les deux moitiés (3, 5) du diaphragme (1) en fonction du signal de sortie des moyens formant capteur (4) détectant une activité naturelle du diaphragme dans au moins une moitié de ce dernier, de manière à obtenir une contraction synchrone des deux moitiés du diaphragme.

6. Dispositif selon la revendication 5, incluant en outre des moyens formant capteur (4) pour détecter l'activité de l'autre moitié du diaphragme.

7. Dispositif selon l'une quelconque des revendications précédentes, dans lequel les moyens formant conducteurs (11) sont adaptés pour être placés contre le diaphragme (1), les électrodes de stimulation (2) étant en contact avec la surface extérieure du diaphragme.

8. Dispositif selon l'une quelconque des revendications précédentes, dans lequel:

a) les moyens formant conducteurs (11) comportent une pluralité d'électrodes espacées (2) placées en contact avec le tissu du diaphragme; et

b) les moyens de stimulation (18) incluent des moyens pour appliquer des salves d'impulsions de stimulation auxdites électrodes avec un retard (figure 10) entre les salves appliquées aux différentes électrodes de manière à obtenir une contraction rythmique du diaphragme.

9. Dispositif selon la revendication 8, dans lequel les salves sont constituées par des impulsions de stimulation possédant une fréquence et/ou une amplitude et/ou une durée variable.

10. Dispositif selon la revendication 8 ou 9, dans lequel les impulsions de stimulation possèdent des formes d'ondes d'impulsions de courant positives et négatives, qui sont sensiblement identiques.

11. Dispositif selon l'une quelconque des revendications précédentes, dans lequel les impulsions de stimulation appliquées aux électrodes de stimulation selon une séquence prédéterminée sont constituées par des trains d'impulsions monophasés ou biphasés ou des trains d'impulsions alternativement monophasés-biphasés, modulés en durée et/ou en amplitude et/ou en fréquence, un retard étant prévu entre les trains d'impulsions arrivant à différentes électrodes, la fréquence respiratoire des trains d'impulsions déterminant le cycle respiratoire.

12. Dispositif selon la revendication 11, dans lequel les enveloppes dudit train d'impulsions incluent des parties croissantes et décroissantes, qui sont rectilignes ou curvilignes.

13. Dispositif selon l'une quelconque des revendications précédentes, comportant en outre:

a) des moyens formant capteur fixés au ou implantés dans le corps du patient pour détecter un paramètre physiologique associé à l'activité respiratoire et développer un signal de commande de l'amplitude respiratoire, possédant une valeur reflétant la force de contraction du diaphragme;

b) des moyens pour appliquer les impulsions de stimulation aux électrodes de stimulation lorsque la valeur dudit signal de commande de l'amplitude respiratoire indique la nécessité d'une assistance respiratoire.

14. Dispositif selon l'une quelconque des revendications 2 à 12, comprenant en outre:

a) des moyens formant capteur adaptés pour être fixés ou implantés sur ou dans le corps d'un patient pour détecter un paramètre physiologique associé à l'activité respiratoire et développer un signal de commande de la fréquence respiratoire, représentant l'intervalle entre des respirations spontanées successives du patient; et

b) des moyens pour appliquer des implusions de stimulation auxdites électrodes de stimulation lorsque la valeur du signal de commande de la fréquence respiratoire représente un intervalle supérieur à un intervalle de référence prédéterminé.

15. Dispositif selon la revendication 14, comprenant en outre:

a) des moyens sensibles auxdits moyens formant capteur pour développer un signal de commande de l'amplitude respiratoire possédant une valeur reproduisant la force de contraction du diaphragme, et

b) des moyens pour appliquer les impulsions de stimulation aux électrodes de stimulation lorsque la valeur dudit signal de commande de l'amplitude respiratoire indique la nécessité d'une assistance respiratoire.

16. Dispositif selon la revendication 13, 14 ou 15, dans lequel lesdits moyens formant capteur (4) détectent l'électrogramme du diaphragme ou dérivent un signal d'impédance obtenu par pléthysmographie à partir d'électrodes espacées implantées sur ou adjacentes au diaphragme ou appliquées sur le thorax du patient et/ou comprennent un capteur de déplacement implanté au voisinage du ou sur le diaphragme (1) pour délivrer un signal en réponse au déplacement du diaphragme.

17. Dispositif selon l'une quelconque des revendications précédentes, comprenant en outre:

a) des moyens formant capteur adaptés pour être implantés dans la circulation sanguine du patient pour la détection d'un paramètre physiologique du sang associé à la respiration et la production d'un signal de commande en réponse à ce paramètre; et

b) des moyens pour appliquer les impulsions de stimulation auxdites électrodes de stimulation lorsque le signal de commande représente un paramètre du sang indicatif de la nécessité d'une respiration.

18. Dispositif selon la revendication 17, dans lequel le capteur détecte le pH, la $pO_2$ et/ou la $pCO_2$ du sang.

FIG. 1

FIG. 2

FIG. 3

FIG. 4

FIG. 5

FIG. 6

FIG. 7

a    b    c

FIG. 8

FIG. 9

FIG. 10

ONE RESPIRATION CYCLE    ONE RESPIRATION CYCLE

STIMULATION
CYCLE

STIMULATION
CYCLE

DELAY

DELAY

TIME

EP 0 190 195 B1

**FIG. II**

**FIG. 12**